(19) Europäisches Patentamt
European Patent Office
Office européen des brevets

(11) Veröffentlichungsnummer: **0 113 030**
**A1**

# (12) EUROPÄISCHE PATENTANMELDUNG

(21) Anmeldenummer: 83111748.6

(22) Anmeldetag: 24.11.83

(51) Int. Cl.³: C 07 C 103/37
C 07 C 103/38, C 07 D 307/14
A 01 N 37/18, A 01 N 39/04
A 01 N 43/08

(30) Priorität: 06.12.82 DE 3245020

(43) Veröffentlichungstag der Anmeldung: 11.07.84 Patentblatt 84/28

(84) Benannte Vertragsstaaten: AT BE CH DE FR GB IT LI NL

(71) Anmelder: CELAMERCK GmbH & Co. KG
Binger Strasse 173
D-6507 Ingelheim am Rhein(DE)

(72) Erfinder: Stransky, Werner, Dr. Dipl.-Chem
Im Hippel 24
D-6535 Gau-Algesheim(DE)

(72) Erfinder: Schröder, Ludwig, Dr. Dipl.-Chem
Frankenstrasse 7
D-6507 Ingelheim(DE)

(72) Erfinder: Mengel, Rudolf, Dr. Dipl.-Chem.
Schützenpfad 22
D-6507 Ingelheim(DE)

(72) Erfinder: Lust, Sigmund, Dr.
Klappacher Strasse 2 f
D-6100 Darmstadt(DE)

(72) Erfinder: Linden, Gerbert, Dr. Dipl.-Landwirt
Turnierstrasse 44
D-6507 Ingelheim(DE)

## (54) Cycloalkenylderivate, ihre Herstellung und Verwendung.

(57) Die neuen Verbindungen der Formel

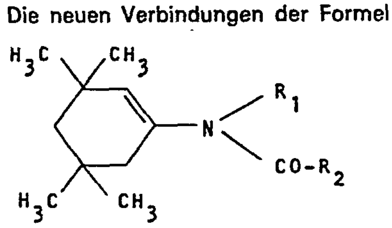

(I),

die in der Beschreibung näher erläutert wird, eignen sich als herbizide Wirkstoffe. Sie können nach üblichen Verfahren synthetisiert werden.

EP 0 113 030 A1

Die Erfindung betrifft neue Cycloalkenylderivate der Formel

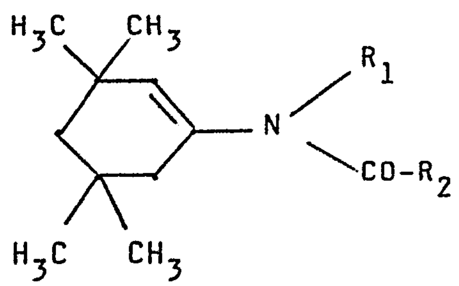

(I),

ihre Herstellung und ihre Verwendung bei der Bekämpfung unerwünschten Pflanzenwuchses.

In der Formel I und auch im folgenden bedeutet

$R_1$ einen Alkenyl- oder Alkinylrest mit bis zu 6 C-Atomen, einen gegebenenfalls phenylsubstituierten Alkylrest mit 1 bis 6 C-Atomen, einen durch 1 bis 3 Sauerstoffatome unterbrochenen, gegebenenfalls zum Ring geschlossenen Alkylrest mit insgesamt 2 bis 8 C-Atomen, einen Cycloalkylrest mit 3 bis 7 C-Atomen oder den Phenylrest,

$R_2$ einen $-CH_2Cl$-, $-CHCl_2$-, $-CH_2OCH_3$- oder $-CH_2OC_6H_5$-Rest, worin die Phenylgruppe ein- oder mehrfach substituiert sein kann.

Die erwähnten Alkyl-, Alkenyl- und Alkinylreste können geradkettig oder verzweigt sein; bevorzugt enthalten sie bis zu 4 C-Atome. Unter "durch Sauerstoff unterbrochenen, gegebenenfalls zum Ring geschlossenen" Alkylresten sind solche zu verstehen, bei denen entweder eine Kette vorliegt, wie in $-CH_2-CH_2-O-C_2H_5$, $-CH_2-CH_2-O-C_2H_4-O-C_2H_5$,

$-C_4H_8-OC_2H_5$ und ähnlichen Gruppen, aber auch solche, bei denen eine Alkylgruppe mit einem sauerstoffunterbrochenen Cycloalkyl verknüpft ist, z.B.

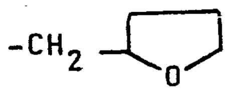 , 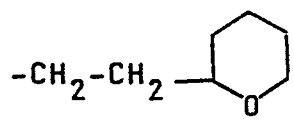

Als Substituenten im Phenylteil des Restes $-CH_2OC_6H_5$ kommen insbesondere ein oder mehrere Halogenatome, z.B. F, Cl, Br, ferner $CH_3$, $CH_3O$, $CH_3S$, $CF_3$ und $NO_2$ in Betracht. Im allgemeinen können 1 bis 3 gleiche oder verschiedene Substituenten vorhanden sein, davon bis zu zwei $CH_3$-, $CH_3O$-, $CH_3S$- und $NO_2$-Gruppen und nicht mehr als eine $CF_3$-Gruppe. $R_2$ steht vor allem für $CH_2Cl$.

Die neuen Verbindungen werden nach an sich bekannten Verfahren hergestellt, insbesondere indem man ein Azomethin der Formel

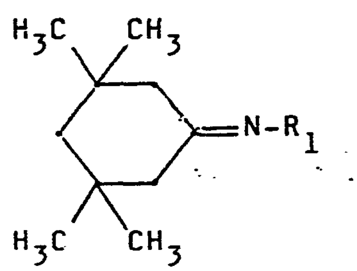 (II)

mit einem Säurehalogenid der Formel

$$X - CO - R_2 \qquad \text{(III)}$$

worin X für Chlor oder auch Brom steht, umsetzt.

Die Umsetzung erfolgt bei Temperaturen zwischen etwa 0°C und der Siedetemperatur des Reaktionsgemischs, im allgemeinen in Lösungsmitteln, die unter den Reaktionsbedingungen hinreichend inert sind; etwa aromatischen Kohlenwasserstoffen wie Toluol, Xylol, Benzol, Pyridin, alkylsubstituierte Pyridine. Falls nicht das Lösungsmittel eine Base ist, setzt man zweckmäßig ein säurebindendes Mittel zu, z.B. tertiäre Amine, Alkalicarbonate.

Die Ausgangsstoffe der Formel II werden in an sich bekannter Weise aus dem Keton

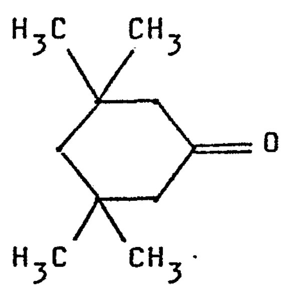

(IV)

und den Aminen der Formel

$$H_2N - R_1 \qquad (V)$$

erhalten.

Die Reaktionskomponenten werden in Lösungsmitteln wie Benzol, Toluol, Xylol in der Wärme umgesetzt, wobei das gebildete Wasser durch einen Wasserabscheider entfernt wird. Niedrigsiedende Amine werden langsam in die siedende Lösung getropft, höhersiedende Amine vorgelegt. Die Reaktion wird durch Säuren katalysiert, z.B. durch p-Toluolsulfonsäure oder Zinkchlorid.

Die Verbindungen der Formel I eignen sich besonders zur Unkrautbekämpfung. Sie können vor und nach dem Auflaufen angewendet werden und wirken in zahlreichen Kulturen selektiv gegen ein breites Spektrum von Unkräutern und Ungräsern, z.B. Amaranthus, Sinapis, Lycopersica, Echinochloa, Avena, Alopecurus, Sorghum, Desmodium. Gute Verträglichkeit findet sich beispielsweise bei Getreide, Mais, Reis, Baumwolle, Soja, Kartoffeln, Bohnen, Zuckerrüben.

Für die Anwendung werden mit üblichen Hilfs- und/oder Trägerstoffen aus den Verbindungen der Formel I anwendungsfertige Mittel hergestellt oder Konzentrate, die vor der Anwendung mit Wasser auf die gewünschte Konzentration verdünnt werden. Als Konzentrate kommen vor allem Lösungs- bzw. Emulsionskonzentrate und Suspensionspulver in Betracht, für die unmittelbare Anwendung z.B. wässerige Spritzbrühen, Stäube, Mikrogranulate. Die Konzentrate können bis zu etwa 95 Gewichtsprozent Wirkstoff enthalten, in den Spritzbrühen ist die Wirkstoffkonzentration im allgemeinen zwischen etwa 0,1 und 2 Gewichtsprozent. Die Aufwandmengen liegen zwischen ca. 0,1 und 6, vorzugsweise 0,3 und 3 kg/ha.

Ein Beispiel für die Formulierung der neuen Verbindungen wird nachstehend gegeben.

Suspensionspulver

Zusammensetzung:

| | |
|---|---|
| Verbindung der Formel I | 300 g |
| Calciumligninsulfonat | 48 g |
| Diisobutylnaphthalinnatrium-sulfonat | 12 g |
| kolloidale Kieselsäure | 180 g |
| Chinafill | 60 g |
| | 600 g |

Das durch Verarbeitung der genannten Komponenten erhaltene Suspensionspulver wird für die Anwendung mit Wasser zu einer Spritzbrühe verrührt, worin der Wirkstoffgehalt ca. 0,1 bis 2 Gewichtsprozent beträgt.

Die Herstellung der Verbindungen der Formel I ist in den nachstehenden Beispielen näher erläutert.

Beispiel 1

N-Allyl-N-[1-(3.3.5.5-tetramethylcyclohexen-1-yl)]-2-chloracetamid

154g (1 mol) 3.3.5.5-Tetramethylcyclohexanon werden in 2 L Toluol gelöst, mit einer Spatelspitze Zinkchlorid versetzt und zum Rückfluß erhitzt. In die siedende Lösung tropft man langsam 68,5g (1,2mol) Allylamin ein und entfernt das entstehende Wasser durch einen Wasserabscheider. Anschließend wird zur Entfernung überschüssigen Amins das Lösungsmittel weitgehend abgezogen. Der Rückstand wird in 1 L absolutem Toluol aufgenommen und unter Eiskühlung tropft man 113 g (1 mol) Chloracetylchlorid zu. Nach einstündigem Rühren läßt man 101 g (1mol) Triethylamin zutropfen, saugt den Niederschlag ab, wäscht das Filtrat mehrmals mit Wasser und zieht nach dem Trocknen das Lösungsmittel ab. Der ölige Rückstand ergibt nach der Destillation 157 g (58 % Ausbeute) N-Allyl-N-[1-(3.3.5.5-tetramethylcyclohexen-1-yl)]-2-chloracetamid vom Siedepunkt 121-129°C bei 0,5 mbar.

Beispiel 2

N-Benzyl-N-[1-(3.3.5.5-tetramethylcyclohexen-1-yl)]-2-chloracetamid

15,4 g (0,1mol) 3.3.5.5-Tetramethylcyclohexanon und 10,8 g (0,1 mol) Benzylamin werden in 200 ml Toluol gelöst, mit einer Spatelspitze p-Toluolsulfonsäure versetzt und zum Rückfluß erhitzt, wobei das entstehende Wasser durch einen Wasserabscheider entfernt wird. Wenn die Wasserabscheidung beendet ist, wird das Lösungsmittel abgezogen,der Rückstand in absolutem Tetrahydrofuran aufgenommen und unter Eiskühlung mit 11,3 g (0,1 mol) Chloracetylchlorid versetzt. Nach einstündigem Rühren tropft man 10,1 g (0,1 mol) Triethylamin zu und saugt den entstandenen Niederschlag ab. Das Filtrat wird eingeengt, der Rückstand in Ether aufgenommen, mit Wasser mehrmals gewaschen und nach dem Trocknen das Lösungsmittel

abgezogen. Die Destillation des Rückstands ergibt 16,6 g (52 % Ausbeute) N-Benzyl-N-[1-(3.3.5.5-tetramethyl-cyclohexen-1-yl)]-2-chloracetamid vom Siedepunkt 152-154°C bei 0,3 mbar.

Entsprechend erhält man die Verbindungen, die in der Tabelle zusammengestellt sind.

Weitere Verbindungen der Formel I

| Nr. | $R_1$ | $R_2$ | physik. Kenndaten |
|---|---|---|---|
| 1 | $-CH_2-CH=CH_2$ | $-CHCl_2$ | Kp.115-120°C/0,8 mbar |
| 2 | $-CH(CH_3)_2$ | $-CH_2Cl$ | Kp.125°C/0,5 mbar |
| 3 | $-CH_3$ | $-CH_2Cl$ | Fp. 58-61°C |
| 4 | $-CH_2-CH=CH_2$ | $-CH_2-O-CH_3$ | Kp.108-112°C/0,6 mbar |
| 5 | $-CH_2-CH=CH_2$ | $-CH_2-O-C_6H_5$ | Kp.153-155°C/0,3 mbar |
| 6 | $-CH(CH_3)_2$ | $-CH_2O-CH_3$ | Kp.110-120°C/0,4 mbar |
| 7 | $-CH(CH_3)_2$ | $-CH_2-O-C_6H_5$ | Fp. 79-82°C |
| 8 | $-CH_2-CH(CH_3)_2$ | $-CH_2Cl$ | Kp.119-127°C/0,4 mbar |
| 9 | $-CH_2CH_2CH_3$ | $-CH_2-O-CH_3$ | Öl |
| 10 | $-CH-C_2H_5$<br>$CH_3$ | $-CH_2Cl$ | Fp. 130-136°C |
| 11 | $-CH_2-CH(CH_3)_2$ | $-CH_2-O-$ 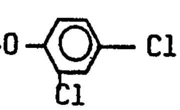 | Fp.86°C |
| 12 | $-CH-C_2H_5$<br>$CH_3$ | $-CH_2-O$ 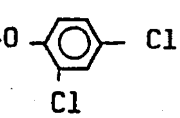 | Öl ber. C 64,06 % H 7,56 % N 3,39 %<br>gef. C 63,74 % H 7,39 % N 3,16 % |
| 13 | $-CH_2-CH_2-O-CH_3$ | $-CH_2Cl$ | Öl ber. C 62,61 % H 9,04 % N 4,86 %<br>gef. C 62,67 % H 9,23 % N 4,96 % |
| 14 | $-CH_2-CH_2-O-CH_3$ | $-CH_2-O$ 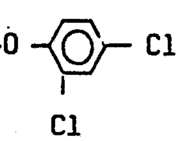 | Öl ber. C 60,86 % H 7,05 % N 3,38 %<br>gef. C 59,97 % H 6,78 % N 3,23 % |
| 15 | $-CH_2-C \equiv CH$ | $-CH_2Cl$ | Kp.134-140°C/0,5 mbar |
| 16 | $-CH_2-CH_2-C_6H_5$ | $-CH_2Cl$ | Öl ber. C 71,92 % H 8,45 % N 4,19 %<br>gef. C 71,86 % H 8,51 % N 4,06 % |

| Nr. | $R_1$ | $R_2$ | physik. Kenndaten |
|---|---|---|---|
| 17 | $-CH_2-$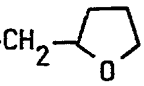 | $-CH_2Cl$ | Öl ber. C 65,05 % H 8,99 % N 4,46 %<br>gef. C 64,79 % H 8,81 % N 4,40 % |
| 18 | $-CH_2-C=CH_2$<br>$CH_3$ | $-CH_2Cl$ | Öl ber. C 67,70 % H 9,23 % N 4,94 %<br>gef. C 67,93 % H 9,51 % N 4,63 % |
| 19 | 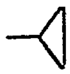 | $CH_2Cl$ | Kp.107-114°C/0,3 mbar |
| 20 | $-C_6H_5$ | $CH_2Cl$ | ber. C 70,68 % H 7,91 % N 4,58 %<br>gef. C 70,86 % H 7,83 % N 4,39 % |

Patentansprüche

1. Verbindungen der Formel

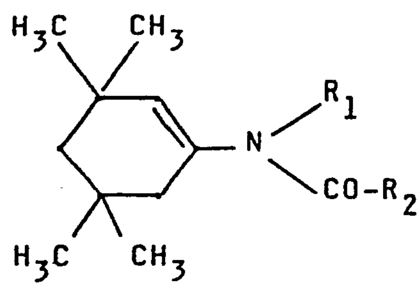

(I)

in der

$R_1$ einen Alkenyl- oder Alkinylrest mit bis zu 6 C-Atomen, einen gegebenenfalls phenylsubstituierten Alkylrest mit 1 bis 6 C-Atomen, einen durch 1 bis 3 Sauerstoffatome unterbrochenen, gegebenenfalls zum Ring geschlossenen Alkylrest mit insgesamt 2 bis 8 C-Atomen, einen Cycloalkylrest mit 3 bis 7 C-Atomen oder den Phenylrest,

$R_2$ einen $-CH_2Cl-$, $-CHCl_2-$, $-CH_2OCH_3-$ oder $-CH_2OC_6H_5-$Rest, worin die Phenylgruppe ein- oder mehrfach substituiert sein kann

bedeutet.

2. Verbindungen der Formel (I), in denen $R_2$ Chlormethyl bedeutet.

3. Verbindungen der Formel (I), in denen $R_1$ einen gegebenenfalls sauerstoffunterbrochenen Alkylrest mit 1 bis 4 C-Atomen oder einen Alkenyl- oder Alkinylrest mit bis zu 4 C-Atomen und $R_2$ Chlormethyl bedeutet.

4. Mittel zur Bekämpfung unerwünschten Pflanzenwachstums, gekennzeichnet durch einen Gehalt an einer Verbindung nach Anspruch 1, 2 oder 3.

5. Verwendung von Verbindungen nach Anspruch 1, 2 oder 3 bei der selektiven Bekämpfung unerwünschten Pflanzenwachstums.

6. Verfahren zur Herstellung von Verbindungen der Formel

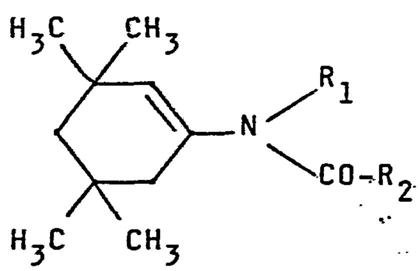

(I),

in der

$R_1$ einen Alkenyl- oder Alkinylrest mit bis zu 6 C-Atomen, einen gegebenenfalls phenylsubstituierten Alkylrest mit 1 bis 6 C-Atomen, einen durch 1 bis 3 Sauerstoffatome unterbrochenen, gegebenenfalls zum Ring geschlossenen Alkylrest mit insgesamt 2 bis 8 C-Atomen, einen Cycloalkylrest mit 3 bis 7 C-Atomen oder den Phenylrest,

$R_2$ einen $-CH_2Cl-$, $-CHCl_2-$, $-CH_2OCH_3-$ oder $-CH_2OC_6H_5-$Rest, worin die Phenylgruppe ein- oder mehrfach substituiert sein kann bedeutet,

dadurch gekennzeichnet, daß man eine Verbindung der Formel

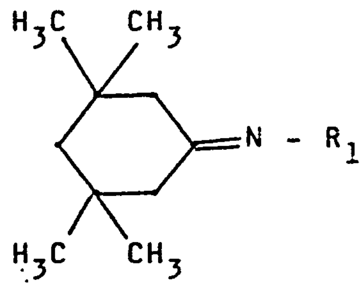

(II)

in der $R_1$ die oben angegebene Bedeutung hat, mit einem Säurehalogenid der Formel

$$X - CO - R_2 \quad (III),$$

in der $R_2$ die oben angegebene Bedeutung hat und X für Chlor oder Brom steht, umsetzt.

7. N-Allyl-N-[1-(3.3.5.5-tetramethylcyclohexen-1-yl)]-2-chloracetamid.

8. N-Isopropyl-N-[1-(3.3.5.5-tetramethylcyclohexen-1-yl)]-2-chloracetamid.

9. N-Propargyl-N-[1-(3.3.5.5-tetramethylcyclohexen-1-yl)]-2-chloracetamid.

Europäisches Patentamt

# EUROPÄISCHER RECHERCHENBERICHT

0113030

Nummer der Anmeldung

EP 83 11 1748

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 013 429 (CHEMISCHE WERKE HÜLS) * Ansprüche; Beispiel 1 * --- | 1,4-6 | C 07 C 103/37<br>C 07 C 103/38<br>C 07 D 307/14<br>A 01 N 37/18<br>A 01 N 39/04<br>A 01 N 43/08 |
| Y | US-A-4 351 667 (J.P. CHUPP) * Ansprüche; Spalten 2,3 * --- | 1,4,5 | |
| A | JOURNAL OF THE CHEMICAL SOCIETY, CHEMICAL COMMUNICATIONS, Nr. 13, 1981, Seiten 692-694 I. NINOMIYA et al.: "Unusual photocyclisation of 1-(N-methyl-1-naphthoylamino)tetramethyl-cyclohexene; X-ray crystal structure of 4b,8,9,10,10a,10b,11,12-octahydro-6,8,8,10-tetramethyl-10,12-methanobenzo[i]-phenanthridin-5(6H)-one" * Seite 693 * ----- | 1 | |
| | | | RECHERCHIERTE SACHGEBIETE (Int. Cl. 3) |
| | | | C 07 C 103/00<br>C 07 D 307/00<br>A 01 N 37/00<br>A 01 N 39/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 13-03-1984 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82